# FASCICULE DE BREVET EUROPEEN

(11) **EP 0 332 604 B1**
(45) Date de publication et mention de la délivrance du brevet: **12.07.1995**
(21) Numéro de dépôt: 89870040.6
(22) Date de dépôt: 09.03.1989
(51) Int. Cl.: C12N 1/00, A23K 1/18

(54) **Aliment pour aquaculture**
Nahrung für Aquakultur
Feed for aquaculture

(30) Priorité: 09.03.1988 FR 8803034
(43) Date de publication de la demande: 13.09.1989
(73) Titulaire: INVE AQUACULTURE, B-9200 Baasrode (BR)
(72) Inventeur: Lavens, Patrick, B-8100 Torhout (BE); Coutteau, Peter, B-8280 Koekelare (BE); Sorgeloos, Patrick, B-9630 Zwalm (BE); Vandamme, Erick, B-9030 Gent (BE)
(74) Mandataire: Van der Stock, Guy

(56) Documents cités:
- FR-A- 2 121 733
- LU-A- 79 882
- US-A- 3 576 643
- CHEMICAL ABSTRACTS, vol. 75, no. 7, 16 août 1971, page 104, résumé no. 45963f, Columbus, Ohio, US; S. YAMAMOTO et al.: "Microbial enzymes active in hydrolyzing yeast cell wall. II. Lysis of mannan layer by some enzyme preparations and reducing agents", & HAKKO KOGAKU ZASSHI 1971, 49(4), 338-42

## Description

La présente invention concerne l'utilisation d'une levure comme aliment en aquaculture.

Plus particulièrement, la présente invention concerne l'utilisation d'une levure comme aliment hautement digestible par les mollusques et crustacés, leurs larves et les alevins, ainsi que par les invertébrés aquatiques qui constituent leurs aliments.

Le développement de l'aquaculture a conduit de nombreuses équipes à étudier l'alimentation des alevins et des larves aquatiques.

L'alimentation de ces animaux a deux sources :
- les algues microscopiques ou micro-algues;
- les invertébrés microscopiques (Artémia, rotifères par exemple) se nourrisant eux-mêmes de micro-algues.

Les micro-algues constituent donc le premier maillon de la chaîne alimentaire applicable en milieu aquatique.

Néamoins, ces algues ne sont pas aisément disponibles. Il faut les cultiver de manière intensive, ce qui contribue à éléver notablement le prix de revient des produits aquacoles.

Il serait donc particulièrement intéressant de pouvoir remplacer les micro-algues par un produit de substitution.

Un tel substitut doit avoir une valeur nutritionnelle élevée et ne pas modifier les caractéristiques de l'eau.

De plus, il doit répondre aux exigences du prédateur, c'est-à-dire avoir une taille lui permettant d'être avalé en une seule fois sans le fracturer (sans pour autant avoir une consistance pulvérulente), être appétant, et digestible.

Des recherches récentes ont abouti à l'élaboration d'aliments microencapsulés (tel CAR de Frippak-Royaume-Uni); mais leur coût très élevé de fabrication et le fait qu'une partie des nutriments s'échappe de la microcapsule font que ces produits sont d'application délicate et limitée.

Un autre substitut des micro-algues est constitué par des protéïnes d'origine unicellulaire (Single Cell Proteïn ou SCP) et plus particulièrement les levures.

Ces produits sont actuellement utilisés dans la formulations de certains aliments, notamment pour le bétail.

Bien que ce type de produit à base de SCP soit l'objet d'un certain engouement depuis ces dernières années, leur utilisation se heurte à des difficultés provenant en particulier de leur digestibilité qui est assez mauvaise. Cette mauvaise digestibilité implique qu'avant leur incorporation aux aliments pour bétail, on fasse éclater les cellules, par broyage ou traitement thermique.

Un tel produit traité est admissible pour nourrir du bétail, mais est incompatible avec l'alimentation d'animaux en milieu aquatique. En effet, l'utilisation d'un tel produit conduit à une pollution importante de l'eau qui est difficilement acceptable.

Des expériences poursuivies par la Demanderesse ont permis de mettre au point des produits tels que celui décrit dans la demande de brevet EP-209510-A qui utilise des cellules de levures non éclatées pour l'alimentation en aquaculture.

Bien que ce produit constitue une amélioration, notamment dans l'alimentation de certaines espèces de crevettes, il est difficilement digéré par les larves d'huitres et par Artemia.

Aucune recherche n'a, jusqu'à présent, abouti à la préparation d'un substitut des micro-algues convenable, c'est-à-dire, présentant les avantages nutritionnels des micro-algues, mais étant plus faciles à obtenir que ces dernières.

Dans l'extrait de Chemical Abstracts, Vol. 75, n° 7, 16 (1972) page 104, résumé n° 45963f, est décrit un procédé d'hydrolyse de levure.

La Demanderesse propose maintenant l'utilisation comme aliment pour aquaculture d'une levure dont les cellules sont traitées pour hydrolyser au moins partiellement la couche externe de la paroi cellulaire sans endommager la paroi elle-même.

L'utilisation, selon l'invention, d'une levure ainsi traitée permet d'obtenir une nourriture hautement digestible, notamment par les mollusques, les crustacés et leurs larves.

Cette utilisation est particulièrement appropriée pour les levures de type hémiascomycète, dont la couche externe contient des ponts disulfures. Plus particulièrement, on préfère utiliser les levures Saccharomyces, de préférence Saccharomyces cerevisiae.

Les essais réalisés avec des réactifs hydrolysant au moins partiellement la couche externe de la paroi cellulaire, soit par voie enzymatique, soit par voie chimique, ont montré que les cellules de levure ainsi traitées présentaient une digestibilité améliorée bien que les cellules traitées gardent leur intrégrité.

De manière particulièrement avantageuse, selon l'invention, ces levures sont accessibles aux enzymes digestives de leurs prédateurs mais dont la paroi reste suffisamment intacte pour que le contenu cellulaire ne se répande pas dans l'eau.

Une telle stabilité de la levure hautement digestible ainsi obtenue selon le procédé empêche la pollution de l'eau par le contenu cellulaire.

La levure utilisée comme aliment pour aquaculture est avantageusement obtenue suivant un procédé comprenant les étapes suivantes :
- optionnellement, lavage de la levure,
- mise de la levure en suspension dans une solution contenant un réactif capable d'hydrolyser au moins partiellement la couche externe de la paroi cellulaire,
- incubation de la suspension,
- optionnellement, récupération des cellules et/ou rinçage
Le lavage de la levure se fait généralement avec de l'eau ou, de manière préférée, au moyen d'une solution aqueuse d'EDTA disodique ou d'un de ses équivalents. La concentration en EDTA disodique est comprise entre 30 et 70 mM, de préférence égale à environ 50 mM. Le pH de la solution d'EDTA est ajusté, de préférence avec du NaOH, à une valeur comprise entre 7 et 12. Ce lavage n'est pas nécessaire mais il apporte souvent un effet bénéfique.

La mise en suspension de la levure s'effectue dans une solution contenant une quantité appropriée d'un réactif capable d'hydrolyser au moins partiellement la couche externe de la paroi cellulaire sans endommager la paroi elle-même. Cette solution peut également contenir de 30 à 70 mM, de préférence 50 mM, d'EDTA disodique, ou un de ses équivalents. Le pH de cette solution est ajusté, de préférence avec du NaOH, à une valeur comprise entre 7 et 12, de préférence d'environ 9. On a constaté que, lorsque l'on utilise un réactif hydrolysant les ponts disulfure, une augmentation du pH permet généralement d'utiliser une plus faible quantité de réactif pour obtenir un résultat équivalent. La solution dans laquelle on procède à la remise en suspension peut être identique à celle utilisée pour le lavage; on peut également ajouter le réactif à la même solution que celle qui avait été utilisée.

Comme réactifs appropriés on peut utiliser des réactifs enzymatiques, par exemple une mannanase. Les enzymes appropriées sont connues de l'homme de l'art, ainsi que les conditions de leur utilisation (pH, concentration, température, durée d'incubation). Cependant, ces réactifs sont généralement coûteux, et on utilise donc de préférence des réactifs chimiques, plus particulièrement ceux capables d'hydrolyser au moins partiellement les ponts disulfure de la couche externe de la paroi cellulaire.

Parmi ces réactifs, on peut utiliser les thiols, de préférence les mercaptoalcools, parmi lesquels le mercaptoethanol et le dithiothréithol sont les plus préférés. On peut envisager d'utiliser les alcoolates ou thioalcoolates de ces composés.

Egalement parmi ces réactifs, on peut utiliser certains acides aminés soufrés, comme la cystéine ou la méthionine, de préférence la cystéine.

Enfin, on peut également utiliser d'autres réducteurs comme des sulfures solubles, de préférence le sulfure de sodium (Na₂S).

La concentration minimale nécessaire pour observer un effet dépend naturellement des conditions d'incubation (pH, température, durée), tandis que l'on évite généralement d'ajouter un excès de réactif chimique pour des raisons évidentes. Ainsi, ces réactifs chimiques sont généralement utilisés à raison de 0,002 à 1 M, de préférence de 0,003 à 0,75 M quand on utilise un thiol et de 0,01 à 0,1 M quand on utilise un acide aminé ou un autre réactif chimique.

On obtient de très bons résultats avec une concentration en mercaptoéthanol de 5% (v/v) à un pH de 9.

La levure en suspension ainsi obtenue est récupérée par centrifugation ou tout autre moyen équivalent et rincée au moins une fois, de préférence trois fois, à l'eau douce ou à l'eau de mer, afin d'éliminer toute trace de réactif.

Les cellules ainsi obtenues peuvent être stockées à une température voisine de 4°C dans l'eau du dernier rinçage pendant au moins une semaine, sans que l'on constate de lyse de la paroi cellulaire.

Les levures traitées selon le procédé peuvent servir telles quelles comme aliment en aquaculture, mais elles sont particulièrement appropriées pour les mollusques et les crustacés, y compris sous leurs différents stades d'évolution.

Elles peuvent aussi servir d'aliments pour des petits invertébrés qui servent eux-mêmes de nourriture vivante pour les animaux cités ci-dessus. C'est le cas, par exemple, pour les Artemia et les rotifères.

Elles peuvent encore être utilisées comme produit de départ pour la réalisation d'un aliment pour aquaculture selon le procédé décrit dans la demande de brevet EP-209510-A. Dans ce cas, on utilise de préférence la cystéine comme réactif, auquel cas l'addition d'huile peut avantageusement se faire directement dans la suspension, à la fin de l'incubation.

Le procédé décrit ci-dessus permet donc de préparer des produits de substitution des micro-algues hautement digestibles par les rotifères, les mollusques et les crustacés et leurs larves. Il s'agit d'un procédé facile à mettre en oeuvre et faisant appel à des matières premières aisées à trouver et peu onéreuses.

D'autres caractéristiques et avantages de la présente invention apparaîtront à la lecture des exemples ci-après.

### EXEMPLE 1

Comparaison entre les résultats d'élevage d'Artemia avec un aliment standard et avec une levure modifiée selon le procédé décrit ci-dessus.

Tous les élevages ont été réalisés dans les mêmes conditions physiques, dans la même eau de mer artificielle.
a) Réalisation de l'eau de mer artificielle
L'eau de mer utilisée pour les élevages est préparée selon la composition connue suivante :

| | |
|---|---|
| - NaCl | 239,0 g |
| - MgCl₂.6H₂O | 108,3 g |
| - CaCl₂ (anhydre) | 11,5 g |
| - SrCl₂.6H₂O | 0,04 g |
| - KCl | 6,82 g |
| - KBr | 0,99 g |
| - NaSO₄.10H₂O | 90,6 g |
| - NaHCO₃ | 0,20 g |
| - NaF | 0,003 g |
| - H₃BO₃ | 0,027 g |
| - eau distillée | 9560 ml |

b) Préparation de la levure
On a appliqué le procédé à la levure de boulanger fraîche Saccharomyces cerevisiae (Gist & Spriritusfabrieken Bruggeman, N.V.). On a mis en oeuvre les étapes suivantes :
- lavage de la levure avec une solution d'EDTA Na₂(50mM) dans l'eau distillée dont on a ajusté le pH à 8 avec du NaOH,
- addition de 2% v/v de mercaptoéthanol à la solution décrite ci-dessus,
- remise en suspension à raison de 500mg/ml (poids mouillé) de la levure dans la solution ainsi obtenue;
- incubation pendant 30 minutes à 30°C,
- récupération des cellules par centrifugation,
- lavage à l'eau de mer, trois fois.

c) Conditions d'élevage
Des cystes secs d'Artemia (souche du Grand Lac Salé), crustacés de la sous-classe des Anostraces, ont été hydratés dans de l'eau de mer artificielle à 25°C sous éclairement constant.
On a récolté les nauplii après 24 heures d'incubation et on les a répartis dans des tubes Falcon Blue Max 1070^{R}, à raison de 25 nauplii par tube, chaque tube contenant 50ml d'eau de mer artificielle aérée au moyen d'une pipette Pasteur.
Les élevages ont duré une semaine. La nourriture a été fournie quotidiennement. Le quatrième jour, avant alimentation, quinze larves survivantes de chaque tube ont été transférées dans des tubes contenant de l'eau de mer artificielle fraîche.
d) Réalisation des élevages
On a divisé l'ensemble des tubes en quatre séries, élevées ensemble dans les mêmes conditions, à l'exception de la nourriture quotidienne :
- la levure obtenue selon le procédé de l'invention, tel que réalisé dans le présent exemple, a été donnée comme aliment pour un élevage d'Artemia,
- un autre élevage d'Artemia a été réalisé avec la même levure mais non traitée,
- un troisième élevage a été réalisé avec du protoplaste de la même souche de Saccharomyces cerevisiae obtenu par des procédures routinières,
- un quatrième élevage (qui peut également servir d'étalon interne pour comparer des élevages successifs) a été réalisé avec une micro-algue unicellulaire vivante Dunalliela tertiolecta connue pour être un aliment adapté à Artemia

e) Résultats
Au 4ème jour (avant transfert) et au 8ème jour, on a déterminé :
- Xₛ :: moyenne du pourcentage de larves survivantes
- Sₛ :: déviation standard sur Xₛ
- X_{c} :: moyenne des longueurs des larves, exprimée en mm
- S_{c} :: déviation standard sur X_{c}
- n :: pour chaque type d'alimentation, nombre de tubes pour lesquels les valeurs précédentes ont été déterminées,
sachant que :
100% de survie au 4ème jour = 25 larves par tube
100% de survie au 8ème jour = 15 larves par tube
La longueur des larves a été mesurée au moyen d'un microscope binoculaire équipé d'un miroir à dessin.
Les résultats sont consignés dans le Tableau 1 ci-après.

| 4ème jour | | | | | | |
|---|---|---|---|---|---|---|
| Aliment | Survie | | | Longueur | | |
| | Xₛ | Sₛ | n | X_{c} | S_{c} | n |
| Protoplaste | 86 | 8 | 29 | 1,77 | 0,10 | 6 |
| Levure brute | 72 | 15 | 11 | 1,31 | 0,06 | 6 |
| Levure traitée | 81 | 7 | 12 | 1,63 | 0,17 | 6 |
| Micro-algue | 94 | 7 | 5 | 1,80 | 0,15 | 5 |

| 8ème jour | | | | | | |
|---|---|---|---|---|---|---|
| Aliment | Survie | | | Longueur | | |
| | Xₛ | Sₛ | n | X_{c} | S_{c} | n |
| Protoplaste | 67 | 14 | 12 | 3,98 | 0,31 | 6 |
| Levure brute | 29 | 8 | 5 | 2,91 | 0,10 | 5 |
| Levure traitée | 68 | 13 | 6 | 4,63 | 0,26 | 6 |
| Micro-algue | 95 | 6 | 5 | 4,24 | 0,18 | 5 |

Les performances du photoplaste de levure sont bonnes, indiquant que le contenu de la cellule de levure est un bon aliment pour

### Artemia.

Les performances de la levure brute sont faibles, à cause de la paroi cellulaire empêchant la digestion du contenu cellulaire.

Les performances de la levure traitée sont presque aussi bonnes que celles du protoplaste sans en avoir les inconvénients (pollution de l'eau).

La levure traitée selon le procédé allie donc les avantages du protoplaste du point de vue nutrition et ceux de la levure brute du point de vue inocuité vis à vis de l'eau.

### EXEMPLE 2

Comparaison entre les résultats d'élevages d'Artemia avec des levures traitées selon différentes variantes du procédé de l'exemple 1.
a) Préparation de la levure
On a appliqué à 2 lots de la même levure que celle utilisée dans l'exemple 1, deux variantes du procédé :
Première variante
- le pH de la solution d'EDTA Na₂ a été ajusté à 9 avec NaOH,
- on a ajouté à cette solution, lors de la remise en suspension de la levure, 5% de mercaptoéthanol (v/v).
Seconde variante
- le pH de la solution d'EDTA Na₂ a été ajusté à 12 avec NaOH,
- on a ajouté à cette solution, lors de la remise en suspension de la levure, 0,02% de mercaptoéthanol (v/v).

b) Réalisation des élevages
Toutes les conditions d'élevage étaient les mêmes que celles décrites dans l'exemple 1.
On a divisé l'ensemble des tubes en cinq séries, élevées ensemble dans les mêmes conditions, à l'exception de la nourriture quotidienne :
- un élevage a été réalisé avec la levure brute,
- un second élevage a été réalisé avec la levure telle que modifiée dans l'exemple 1,
- un troisième élevage a été réalisé avec la levure telle que modifiée selon la première variante décrite ci-dessus,
- un quatrième élevage a été réalisé avec la levure telle que modifiée selon la seconde variante décrite ci-dessus,
- enfin, un élevage témoin a été réalisé avec la micro-algue utilisée dans l'exemple 1.

c) Résultats
Les résultats de survie et de croissance au 4ème jour (avant transfert) et au 8ème jour sont regroupés dans le tableau 2 ci-après :

| 4ème jour | | | | | | |
|---|---|---|---|---|---|---|
| Aliment | Survie | | | Longueur | | |
| | Xₛ | Sₛ | n | X_{c} | S_{c} | n |
| Levure brute | 85 | 7 | 6 | (1,16) | - | - |
| Levure traitée selon l'exemple 1 | 96 | 5 | 6 | (1,82) | - | - |
| Levure traitée selon la 1ère variante | 95 | 5 | 6 | (1,87) | - | - |
| Levure traitée selon la 2ème variante | 99 | 2 | 6 | (1,49) | - | - |
| Micro-algue | 98 | 2 | 5 | (2,04) | - | - |

| 8ème jour | | | | | | |
|---|---|---|---|---|---|---|
| Aliment | Survie | | | Longueur | | |
| | Xₛ | Sₛ | n | X_{c} | S_{c} | n |
| Levure brute | 31 | 17 | 6 | 1,69 | 0,22 | 6 |
| Levure traitée selon l'exemple 1 | 66 | 15 | 6 | 4,76 | 0,25 | 6 |
| Levure traitée selon la 1ère variante | 74 | 7 | 6 | 4,91 | 0,24 | 6 |
| Levure traitée selon la 2ème variante | 67 | 12 | 6 | 4,68 | 0,13 | 6 |
| Micro-algue | 93 | 9 | 5 | 4,37 | 0,30 | 5 |

On a constaté de meilleures performances pour la levure traitée selon les modalités décrites dans l'exemple 2, en particulier dans le cas de la seconde variante qui met en oeuvre des concentrations très faibles en mercaptoéthanol à un pH élevé.

### EXEMPLE 3

Comparaison entre les résultas d'élevage d'Artemia avec des levures traitées avec des acides aminés et avec des réducteurs.
a) Préparation de la levure
On a appliqué à 2 lots de la même levure que celle utilisée dans l'exemple 1, deux variantes du procédé :
Première variante
- on a ajouté à la solution d'EDTA Na₂, de la cystéine à une concentration de 0,1 M,
- on a ajusté le pH de la solution à 8, lors de la remise en suspension de la levure.
Seconde variante
- le pH de la solution d'EDTA Na₂ a été ajusté à 9 avec NaOH,
- on a ajouté à cette solution, lors de la remise en suspension de la levure, 0,1 M de sulfure de sodium (Na₂S).

b) Réalisation des élevages
Toutes les conditions d'élevage étaient les mêmes que celles décrites dans l'exemple 1.
On a divisé l'ensemble des tubes en cinq séries, élevées ensemble dans les mêmes conditions, à l'exception de la nourriture quotidienne :
- un élevage a été réalisé avec la levure telle que modifiée dans la première variante de l'exemple 2,
- un deuxième élevage a été réalisé avec la levure telle que modifiée selon la première variante du présent exemple,
- un troisième élevage a été réalisé avec la levure telle que modifiée selon la seconde variante du présent exemple.

c) Résultats

| 4ème jour | | | | | | |
|---|---|---|---|---|---|---|
| Aliment : levure traitée | Survie | | | Longueur | | |
| | Xₛ | Sₛ | n | X_{c} | S_{c} | n |
| selon l'exemple 2 (variante 1) | 97 | 6 | 6 | (1,49) | - | - |
| selon l'exemple 3 (variante 1) | 91 | 5 | 6 | (1,76) | - | - |
| selon l'exemple 3 (variante 2) | 95 | 4 | 6 | (1,58) | - | - |

| 8ème jour | | | | | | |
|---|---|---|---|---|---|---|
| Aliment : levure traitée | Survie | | | Longueur | | |
| | Xₛ | Sₛ | n | X_{c} | S_{c} | n |
| selon l'exemple 2 (variante 1) | 76 | 11 | 6 | 4,17 | 0,36 | 6 |
| selon l'exemple 3 (variante 1) | 70 | 12 | 6 | 4,07 | 0,65 | 6 |
| selon l'exemple 3 (variante 2) | 41 | 14 | 6 | 4,61 | 0,27 | 6 |

En l'absence d'élevage témoin réalisé en parallèle avec la micro-algue utilisée dans les exemples précédents, les résultats de cet exemple ne doivent être comparés qu'entre eux. Ils montrent notamment que l'utilisation de cystéine est préférable à celle de sulfure de sodium.

## Revendications

1. Utilisation comme aliment pour aquaculture d'une levure dont les cellules sont traitées pour hydrolyser au moins partiellement la couche externe de la paroi cellulaire sans endommager la paroi elle-même.

2. Utilisation suivant la revendication 1, caractérisée en ce que la levure précitée est du type hemiascomycète.

3. Utilisation suivant la revendication 1 ou 2, caractérisée en ce que la levure précitée est une levure Saccharomyces.

4. Utilisation suivant l'une quelconque des revendications 1 à 3, caractérisée en ce que les cellules de levure sont traitées à l'aide d'un réactif qui hydrolyse au moins partiellement les ponts disulfure.

5. Utilisation suivant l'une quelconque des revendications 1 à 4, caracterisée en ce que l'hydrolyse est réalisée enzymatiquement.

6. Utilisation suivant l'une quelconque des revendications 1 à 5, caractérisée en ce que l'hydrolyse est réalisée chimiquement à l'aide d'un réactif choisi parmi les composés ayant au moins une fonction thiol, les sulfures solubles, et leurs mélanges.

7. Utilisation suivant l'une quelconque des revendications 1 à 6, caractérisée en ce que l'hydrolyse est réalisée à l'aide d'un réactif choisi parmi le mercaptoéthanol, le dithiothréitol, la cystéine, le sulfure de sodium, et leurs mélanges.

8. Utilisation comme aliment pour aquaculture d'une levure ayant subi les étapes de traitement suivants :
- mise de la levure en suspension dans une solution aqueuse contenant un réactif choisi parmi les composés ayant au moins une fonction thiol, les sulfures solubles, et leurs mélanges,
- incubation de la suspension.

9. Utilisation suivant la revendication 8, caractérisée en ce que la levure a subi en plus une étape préalable de lavage de la levure et/ou une étape ultérieure de récupération des cellules de levure et/ou une étape ultérieure de rinçage des cellules de levure.

10. Utilisation suivant l'une ou l'autre des revendications 8 et 9, caractérisée en ce que la levure traitée est du type hémiascomycète.

11. Utilisation suivant l'une quelconque des revendications 8 à 10, caractérisée en ce que la levure traitée est une levure Saccharomyces.

12. Utilisation suivant l'une quelconque des revendications 8 à 11, caractérisée en ce que le réactif est choisi parmi le mercaptoéthanol, le dithiothréitol, la cystéine, le sulfure de sodium, et leurs mélanges.

13. Utilisation suivant l'une quelconque des revendications 8 à 12, caractérisée en ce que la levure est mise en suspension à raison de 300 à 700 mg/ml (poids mouillé) dans une solution aqueuse ayant un pH compris entre 7 et 12 et contenant de 0,0002 à 1 M de réactif.

14. Utilisation suivant l'une quelconque des revendications 8 à 13, caractérisée en ce que le réactif est utilisé à raison de 0,003 à 0,75 M s'il s'agit d'un thiol ou de 0,01 à 0,1 M s'il s'agit d'un acide aminé ou d'un autre réactif.

15. Utilisation suivant l'une quelconque des revendications 8 à 14, caractérisée en ce que l'incubation est réalisée à une température comprise entre 25°C et 35°C pendant une durée comprise entre 15 et 45 minutes.

## Claims

1. Use as a feed for aquaculture of a yeast the cells of which are treated so as to hydrolyse at least partially the outer layer of the cell wall without damaging the wall itself.

2. Use according to claim 1, characterized in that the above-mentioned yeast is of the hemiascomycetous type.

3. Use according to claim 1 or claim 2, characterized in that the above-mentioned yeast is a saccharomyces yeast.

4. Use according to any one of claims 1 to 3, characterized in that the yeast cells are treated with a reagent which at least partially hydrolyses the disulphide bridges.

5. Use according to any one of claims 1 to 4, characterized in that the hydrolysis is effected enzymatically.

6. Use according to any one of claims 1 to 5, characterized in that the hydrolysis is effected chemically by means of a reagent selected from compounds having at least one thiol group, soluble sulphides and mixtures thereof.

7. Use according to any one of claims 1 to 6, characterized in that the hydrolysis is effected by means of a reagent selected from mercaptoethanol, dithiothreitol, cysteine, sodium sulphide and mixtures thereof.

8. Use as a feed for aquaculture of a yeast which has been subjected to the following treatment stages:
- putting the yeast in suspension in an aqueous solution containing a reagent selected from compounds having at least one thiol group, soluble sulphides and mixtures thereof,
- incubation of the suspension.

9. Use according to claim 8, characterized in that the yeast has been additionally subjected to a prior yeast washing stage and/or a subsequent yeast cell recovery stage and/or a subsequent yeast cell rinsing stage.

10. Use according to either one of claims 8 and 9, characterized in that the treated yeast is of the hemiascomycetous type.

11. Use according to any one of claims 8 to 10, characterized in that the treated yeast is a saccharomyces yeast.

12. Use according to any one of claims 8 to 11, characterized in that the reagent is selected from mercaptoethanol, dithiothreitol, cysteine, sodium sulphide and mixtures thereof.

13. Use according to any one of claims 8 to 12, characterized in that the yeast is put in suspension in an amount of from 300 to 700 mg/ml (wet weight) in an aqueous solution with a pH between 7 and 12 and containing from 0.0002 to 1 M of reagent.

14. Use according to any one of claims 8 to 13, characterized in that the reagent is used in an amount of from 0.003 to 0.75 M if it is a thiol or of from 0.01 to 0.1 M if it is an amino acid or another reagent.

15. Use according to any one of claims 8 to 14, characterized in that incubation is effected at a temperature between 25°C and 35°C for a period between 15 and 45 minutes.

## Patentansprüche

1. Verwendung einer Hefe als Aquakulturnahrung, deren Zellen behandelt werden, um mindestens teilweise die äußere Schicht der Zellwand zu hydrolysieren, ohne die Wand selbst zu beschädigen.

2. Verwendung nach Anspruch 1, **dadurch gekennzeichnet**, daß die obenerwähnte Hefe eine Hefe vom Hemiascomyceten-Typ ist.

3. Verwendung nach Anspruch 1 oder 2, **dadurch gekennzeichnet**, daß die obenerwähnte Hefe eine Saccharomyces-Hefe ist.

4. Verwendung nach einem der Ansprüche 1 bis 3, **dadurch gekennzeichnet**, daß die Hefezellen mit einem Reagenz behandelt werden, welches mindestens teilweise die Disulfidbrücken hydrolysiert.

5. Verwendung nach einem der Ansprüche 1 bis 4, **dadurch gekennzeichnet**, daß die Hydrolyse enzymatisch durchgeführt wird.

6. Verwendung nach einem der Ansprüche 1 bis 5, **dadurch gekennzeichnet**, daß die Hydrolyse chemisch mit einem Reagenz durchgeführt wird, das gewählt wird aus Verbindungen mit mindestens einer Thiolfunktion, löslichen Sulfiden und ihren Gemischen.

7. Verwendung nach einem der Ansprüche 1 bis 6, **dadurch gekennzeichnet**, daß die Hydrolyse mit einem Reagenz durchgeführt wird, das gewählt wird aus Mercaptoethanol, Dithiothreitol, Cystein, Natriumsulfid und Gemischen davon.

8. Verwendung einer Hefe als Aquakulturnahrung, welche den folgenden Behandlungsschritten unterworfen wurde:
Suspendieren der Hefe in einer wäßrigen Lösung, welche ein Reagenz enthält, das gewählt wird aus Verbindungen mit mindestens einer Thiolfunktion, löslichen Sulfiden und Gemischen davon,
Inkubation der Suspension.

9. Verwendung nach Anspruch 8, **dadurch gekennzeichnet**, daß die Hefe zusätzlich einem vorangegangenen Schritt des Waschens der Hefe und/oder einem späteren Schritt der Rückgewinnung der Hefezellen und/oder einem späteren Schritt das Spülens der Hefezellen unterworfen wurde.

10. Verwendung nach einem der Ansprüche 8 oder 9, **dadurch gekennzeichnet**, daß die behandelte Hefe eine Hefe vom Hemiascomyceten-Typ ist.

11. Verwendung nach einem der Ansprüche 8 bis 10, **dadurch gekennzeichnet**, daß die behandelte Hefe eine Saccharomyces-Hefe ist.

12. Verwendung nach einem der Ansprüche 8 bis 11, **dadurch gekennzeichnet**, daß das Reagenz gewählt wird aus Mercaptoethanol, Dithiothreitol, Cystein, Natriumsulfid und Gemischen davon.

13. Verwendung nach einem der Ansprüche 8 bis 12, **dadurch gekennzeichnet**, daß die Hefe in einem Verhältnis von 300 bis 700 mg/ml (Feuchtgewicht) in einer wäßrigen Lösung mit einem pH zwischen 7 und 12, welche 0,0002 bis 1 M Reagenz enthält, suspendiert wird.

14. Verwendung nach einem der Ansprüche 8 bis 13, **dadurch gekennzeichnet**, daß das Reagenz in einem Verhältnis von 0,003 bis 0,75 M verwendet wird, wenn es sich um ein Thiol handelt, oder in einem Verhältnis von 0,01 bis 0,1 M verwendet wird, wenn es sich um eine Aminosäure oder ein anderes Reagenz handelt.

15. Verwendung nach einem der Ansprüche 8 bis 14, **dadurch gekennzeichnet**, daß die Inkubation bei einer Temperatur zwischen 25°C und 35°C während eines Zeitraums zwischen 15 und 45 min durchgeführt wird.
